(19) Europäisches Patentamt
European Patent Office
Office européen des brevets

(11) **EP 0 853 612 B1**

(12) # FASCICULE DE BREVET EUROPEEN

(45) Date de publication et mention de la délivrance du brevet:
**14.06.2000 Bulletin 2000/24**

(21) Numéro de dépôt: **96930204.1**

(22) Date de dépôt: **04.09.1996**

(51) Int Cl.[7]: **C07C 281/02**, C07D 209/16, C07C 317/48, C07D 233/64

(86) Numéro de dépôt international:
**PCT/FR96/01349**

(87) Numéro de publication internationale:
**WO 97/09303 (13.03.1997 Gazette 1997/12)**

(54) **DERIVES D'ALFA-HYDRAZINOACIDES ET PROCEDE POUR LES FABRIQUER**

ALPHA-HYDRAZINOSÄURE-DERIVATE UND VERFAHREN ZU IHRER HERSTELLUNG

ALPHA-HYDRAZINO ACID DERIVATIVES AND METHOD FOR MAKING SAME

(84) Etats contractants désignés:
**BE CH DE ES FR GB IT LI NL**

(30) Priorité: **07.09.1995 FR 9510685**

(43) Date de publication de la demande:
**22.07.1998 Bulletin 1998/30**

(73) Titulaire: **CENTRE NATIONAL DE LA RECHERCHE SCIENTIFIQUE
75794 Paris Cédex 16 (FR)**

(72) Inventeurs:
- **COLLET, André
F-69006 Lyon (FR)**
- **VIDAL, Joelle
F-69007 Lyon (FR)**
- **HANNACHI, Jean-Christophe
F-69003 Lyon (FR)**
- **GUY, Laure
F-69100 Villeurbanne (FR)**

(74) Mandataire: **Texier, Christian et al
Cabinet Regimbeau,
26, Avenue Kléber
75116 Paris (FR)**

(56) Documents cités:
- **TETRAHEDRON LETT. (1993), 34(43), 6859-62, XP002003006 NIEDERER, DANIEL A. ET AL: "Amination with N-benzyloxycarbonyl-3-phenyloxaziridine as a route to sensitive chiral.alpha.-hydrazino acids: synthesis of L-hydrazino serine" cité dans la demande**
- **TETRAHEDRON LETT. (1975), (31), 2701-4, XP002003007 ACHIWA, KAZUO ET AL: "Hydrazino acids and peptides. New general synthesis of L-.alpha.-hydrazino acids from L-.alpha.-amino acids" cité dans la demande**
- **CHEMICAL ABSTRACTS, vol. 87, no. 1, 4 Juillet 1977 Columbus, Ohio, US; abstract no. 5648, YAMADA, SHUNICHI ET AL: ".alpha.-Hydrazinocarboxylic acids" XP002003008 cité dans la demande & JP 51 138 602 A (JAPAN)**
- **JOURNAL F. PRAKT. CHEMIE, vol. 314, no. 5-6, 1972, pages 735-750, XP000570141 H. NIEDRICH ET AL.: "N.alpha.-Acylierung von Hydrazinoessigsäure-Derivaten und Synthese von Eledoisin-Octapeptiden mit N.alpha.-acylierter Hydrazinoessigsäure anstelle von Asparagin und Glycin" cité dans la demande**
- **J. CHEM. SOC., PERKIN TRANS. 1 (1987), (4), 885-97, XP002003005 LAWTON, GEOFFREY ET AL: "Regioselectivity in the photochemical ring contraction of 4-diazopyrazolidine-3,5-diones to give aza-.beta.-lactams"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention concerne à titre de produits industriels nouveaux des dérivés d'α-hydrazinoacides protégés ; elle se rapporte également à un procédé pour la préparation de tels composés.

## Art antérieur

Les acides α-hydrazinés sont des analogues des acides α-aminés dans lesquels on a remplacé la fonction amine par une fonction hydrazine.

Lorsqu'on veut utiliser ces acides α -hydrazinés en synthèse et en particulier les incorporer à la place d'un acide aminé dans la chaîne d'un peptide pour former soit un hydrazinopeptide ----CO-NHNH-C--- soit un aminopeptide ----CO-N($NH_2$)-C----, la présence des deux fonctions azotées dans ceux-ci entraîne lors des réactions de couplage peptidique des problèmes de régiosélectivité ainsi que des réactions parasites et des réarrangements moléculaires non souhaités.

Pour éliminer ces inconvénients, il est nécessaire qu'au minimum l'un des deux atomes d'azote de l'hydrazinoacide puisse être temporairement neutralisé par un groupe protecteur GP.

Dans les documents [Tetrahedron Lett. 32, 2765 (1991); J. Prakt. Chem. 314, 735 (1972); J. Prakt. Chem. 316, 729 (1974); J. Prakt. Chem. 314, 751 (1972)] on a introduit de façon directe un groupe GP sur un acide α-hydraziné, et on aboutit le plus souvent à un mélange de produits Nα et Nβ protégés qu'il faut ensuite séparer et purifier.

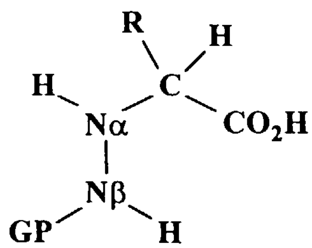

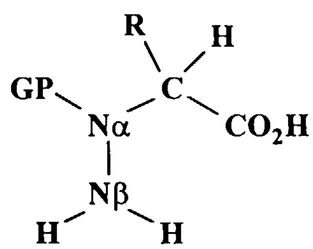

Les inventeurs ont décrit dans les documents [J. Chem. Soc., Chem. Commun. 435 (1991); J. Org. Chem. 58, 4791 (1993)] comment on peut préparer de façon exclusive les composés Nβ protégés en faisant appel à l'amination électrophile d'un acide aminé par une oxaziridine. Dans le cas où GP est Boc (terbutoxycarbonyle), cette méthode donne des rendement médiocres en raison de la formation d'oxazolidinone qui se produit aux dépens du produit désiré au cours de l'isolement. Les rendements sont meilleurs si GP est Moc (méthoxycarbonyle) mais ce groupe se clive dans des conditions trop dures, incompatibles avec la synthèse peptidique.

Dans le document [Tetrahedron 44, 5525 (1988)] on décrit la préparation de composés portant deux groupes protecteurs identiques (Boc) sur les azotes Nα et Nβ. Dans le document [New. J. Chem. 13, 849 (1989)] l'un des coinventeurs utilise le radical acétyle (Ac) pour protéger les deux azotes.

Les composés de formule générale

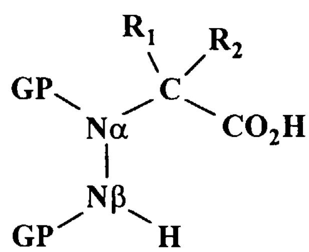

dans lesquels les deux groupes protecteurs GP sont identiques ne sont pas facilement utilisables en synthèse peptidique, d'une part, parce que ces groupes ne peuvent pas être clivés sélectivement l'un par rapport à l'autre (libérant ainsi les deux groupes azotés) et d'autre part, en ce qui concerne le groupe Ac, parce que son clivage nécessite des conditions drastiques incompatibles avec la synthèse peptidique.

Dans les documents [J. Prakt. Chem. 314, 735 (1972); Chem. Ber. 99, 3914 (1966); Tetrahedron Lett. 31, 2701 (1975); Brevet Jap. 76138602-761130 (1976) ; Tetrahedron Lett. 34, 6859 (1993)] on décrit des composés de

formule générale :

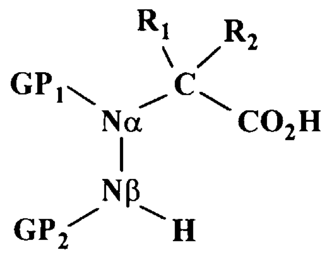

portant deux groupes protecteurs différents $GP_1$ et $GP_2$. Toutefois, si l'un des groupes proposés est classique (Boc, Z (benzyloxycarbonyle), ou benzyle), l'autre (Ac ou Tos) est impropre à la synthèse peptidique en raison des conditions drastiques nécessaires à son clivage.

Dans le document [J. Chem. Soc. Perkin Trans. 1 (1987), (4), 885-97], on a décrit deux dérivés de l'acide hydrazino-acétique, utilisés comme intermédiaires dans la synthèse d'hydrazines. Les enseignements de ce document ne sont pas néanmoins pertinents à l'encontre de l'invention.

L'invention pallie les inconvénients soulignés précédemment. Elle vise à titre de produits industriels nouveaux des dérivés d'hydrazinoacides dans lesquels les deux fonctions amines portent des groupes protecteurs orthogonaux, c'est-à-dire qui peuvent être manipulés indépendamment l'un de l'autre, par exemple lors d'une synthèse peptidique.

## Brève description de l'invention

L'invention concerne à titre de produits industriels nouveaux des dérivés d'hydrazinoacides de formule générale :

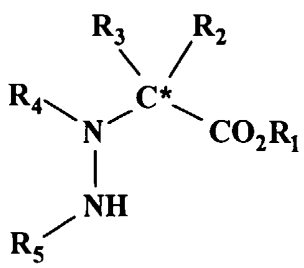

dans laquelle :

- $R_1$, $R_2$, $R_3$ désignent l'hydrogène ou un radical carboné,
- lorsque $R_2$ et $R_3$ sont différents, C* désigne un carbone asymétrique de configuration L, D ou DL,
- $R_4$ et $R_5$ désignent un groupe protecteur,

caractérisé :

- en ce que $R_4$ désigne un radical benzyle $ArCH_2$ de formule :

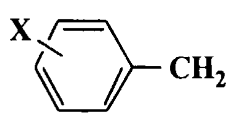

  - où Ar désigne un radical phényle ou phényle substitué par un ou plusieurs groupes X ;
  - où X désigne l'hydrogène, un halogène, un radical nitro ou un radical alkyle ;

- et en ce que $R_5$ désigne un groupe Y-O-CO, dans lequel Y désigne un radical carboné différent de $R_4$.

En d'autres termes, l'invention concerne des acides α-hydrazinés dont les deux groupements azotés Nα et Nβ portent des groupements protecteurs de nature différente, notamment benzyle ($PhCH_2$) sur le Nα, et terbutoxycarbonyle (Boc) ou fluorénylméthoxycarbonyle (Fmoc) sur le Nβ, groupes dont le caractère othogonal permet de les manipuler indépendamment l'un de l'autre.
Ces composés sont directement utilisables dans les synthèses de pseudopeptides par des méthodes classiques en phase solide ou en phase liquide, et sont également utilisables dans le contexte des méthodes de synthèse combinatoire actuellement en plein développement.
Avantageusement, en pratique :

- $R_1$ est l'hydrogène ou un groupe alkyle ou benzyle ;
- $R_2$ désigne l'hydrogène ou un groupe alkyle ;
- $R_3$ désigne l'hydrogène ou une chaîne latérale (éventuellement protégée) des acides aminés protéogéniques naturels, un groupe aromatique tel que le phényle ou le para-hydroxyphényle, un alkyle primaire, secondaire ou tertiaire.

De préférence, $R_3$ désigne outre l'hydrogène, les chaînes latérales (éventuellement protégées) des acides aminés protéogéniques, c'est-à-dire des énantiomères purs de la série L, ainsi que leurs antipodes de la série D et les mélanges racémiques ou partiellement dédoublés DL.
Avantageusement, selon l'invention, dans le groupe protecteur $R_4$ lié à l'azote Nα, X est égal à H.
De même, toujours selon l'invention, dans le groupe protecteur $R_5$ lié à l'azote terminal Nβ, Y est avantageusement le groupe terbutyle (Y-O-CO est Boc) ou le groupe 9-fluorénylméthyle (Y-O-CO est Fmoc).
L'invention concerne également un procédé pour la préparation de ces composés.
Avantageusement, les composés pour lesquels $R_5$ est Boc ou Fmoc sont obtenus de façon directe par réaction des acides α-aminés-N-benzylés correspondants (dont la préparation est triviale) avec une oxaziridine aminante, dont l'azote porte un groupe Boc ou Fmoc, à l'instar de ce que les auteurs ont décrit dans les documents suivants [J. Org. Chem. 58, 4791 (1993); Tetrahedron Lett. 36, 1439 (1995)].
Dans cette réaction, l'ensemble du fragment N-Boc (ou N-Fmoc) de l'oxaziridine est transféré sur l'azote de l'acide α-aminé-N-benzylé pour conduire au composé recherché. La réaction s'effectue dans le dichlorométhane (ou dans d'autres solvants comme l'éther ou le chloroforme) à une température comprise entre -20 °C et +20 °C selon les cas (le plus souvent 0 °C).
Parmi les caractéristiques importantes de la réaction, on doit relever :

a) l'acide α-aminé-N-benzylé, que l'on prépare par des méthodes classiques, est rendu soluble dans le solvant de la réaction par transformation in situ en sel d'ammonium quaternaire (par ex., sel de tétraéthylammonium) ou, dans certains cas, en sel de métal alcalin (par ex., sel de sodium) ;
b) les exemples 1 à 11 décrits ci-dessous utilisent comme réactif d'amination la N-Boc-3-(4-cyanophényl)oxaziridine (ou BCPO), pour aboutir aux composés cherchés avec $R_5$=Boc. D'autres oxaziridines de la même famille peuvent être utilisées, en particulier la N-Boc-3-(2,4-dichlorophényl)oxaziridine dont le coût est moins élevé ;
c) les composés dans lesquels $R_5$ est Fmoc peuvent être obtenus de façon similaire à ceux où $R_5$ est Boc, en utilisant la N-Fmoc-3-phényloxaziridine ; plus avantageusement, il peuvent être préparés indirectement à partir des composés où $R_5$ est Boc par déprotection du Nβ suivie de reprotection par Fmoc-Cl (voir l'exemple 12 ci après) ;
d) les acides α-aminé-N-benzylés de la série L conduisent aux composés recherchés de la même configuration L avec conservation de la pureté énantiomérique. Les acides α-aminé-N-benzylés de la série D conduisent similairement aux composés cherchés de la série D, et les acides α-aminé-N-benzylés DL (racémiques) conduisent similairement aux composés racémiques ;
e) certains composés dont les caractéristiques physiques peuvent être défavorables (hygroscopicité) sont isolés commodément sous forme de sels d'amines (par exemple sels de dicyclohexylamine).

La manière dont l'invention peut être réalisée et les avantages qui en découlent ressortiront mieux des exemples de réalisation qui suivent.

## Manières de réaliser l'invention

Dans un ballon de 10 ml, on place 1 mmol d'acide aminé N-benzylé, puis 0,667 ml (1 mmol) d'une solution méthanolique 1,5 M d'hydroxyde de tétraéthylammonium. On laisse environ 15 mn à température ambiante sous agitation jusqu'à ce que la solution devienne limpide. Le méthanol est évaporé, et remplacé par 3 ml de dichlorométhane. À cette solution, refroidie à 0 °C dans un bain de glace, on ajoute goutte à goutte 246 mg de N-tert-butoxycarbonyl-3-(4-cyanophényl)-oxaziridine (1 mmol) dissoute dans 2 ml de dichlorométhane. Le ballon est bouché hermétiquement

et est mis au réfrigérateur pendant12 heures.

Traitement : On évapore le solvant, et reprend le résidu huileux par 50 ml d'eau contenant 12 mg de soude (0,3 mmol). Après 30 mn d'agitation, il se forme un précipité de paracyanobenzaldéhyde que l'on sépare par filtration. La phase aqueuse restante est salée et lavée par 7 fois 10 ml l'éther, puis acidifiée à pH 3 avec 177 mg d'hydrogénosulfate de potassium (1,3 mmol). On obtient une phase laiteuse que l'on extrait par deux fois 20 ml de dichlorométhane (ou d'éther). Après séchage sur sulfate de sodium et évaporation du solvant on obtient le produit cherché le plus souvent sous la forme d'un solide dont la pureté chimique est très bonne. Dans certains cas le produit peut être recristallisé dans un solvant approprié (par ex., mélanges pentane / oxyde d'isopropyle). Certains produits ont été aussi isolés sous forme de sels d'amines ou de sodium.

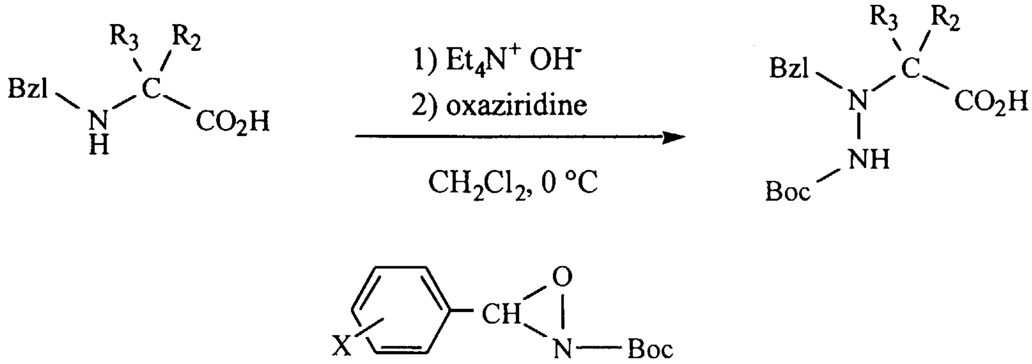


Exemple 1 : Préparation de $N^{\alpha}$-benzyl-$N^{\beta}$-Boc-(L)-hydrazinoalanine (1a)

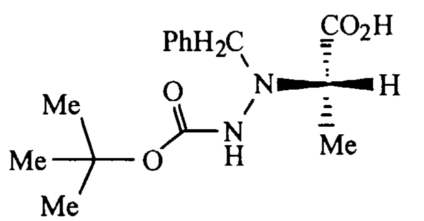

(L)-(1a)

Selon le mode opératoire type, 1,435 g de N-benzyl-(L)-alanine (8 mmol) donnent 2,09 g (88%) de composé (1a) solide. *Caractéristiques physiques :* F 117 °C ; $[\alpha]_D^{25}$ +22,8 (c 1,14 ; MeOH) ; RMN ($CDCl_3$) δ : 1,32 (s, 9H, Boc) 1,38 (d, J = 7 Hz, 3H, Me) 3,64 (q, J = 7 Hz, 1H, $CH^{\alpha}$) 3,95 (s, 2H, $CH_2$ Bzl) 5,85 (s, 1H, NH) 7,26-7,36 (m, 5H, arom). Le sel de dicyclohexylamine de (1a) a un point de fusion F 170 °C (décomposition), et une rotation spécifique $[\alpha]_D^{25}$ +43,7 (c =1,33 ; MeOH). RMN ($CDCl_3$) δ : 1,22-1,67 (m, 24H, Boc + Cy +Me) 1,79 (m, 4H, Cy) 2,02 (m, 4H, Cy) 2,93 (m, 2H, Cy) 3,38 (q, J = 7 Hz, 1H, $CH^{\alpha}$) 4,01 (s, 2H, $CH_2$ Bzl) 7,23 et 7,39 (m, 5H, arom) Cy = cyclohexyle. *Analyse élémentaire* [$C_{15}H_{22}N_2O_4$ + $C_{12}H_{23}N$ + 0,25 $H_2O$], calc (%) C 67,54 ; H 9,55 ; N 8,75; trouvé C 67,30 ; H 9,52; N 8,67.

Exemple 2 : Préparation de N$^{\alpha}$-benzyl-N$^{\beta}$-Boc-(L)-hydrazinovaline (1b)

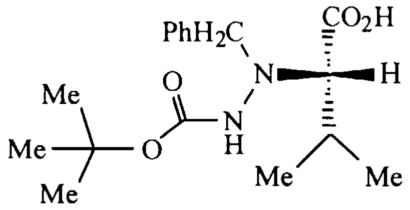

(L)-(1b)

Selon le mode opératoire type, en partant de 207 mg (1 mmol) de N-benzyl-(L)-valine, on obtient 220 mg (68%) de (1b) sous la forme d'un solide, F 117 °C (décomposition), $[\alpha]_D^{25}$ +25,6 (c=1,19; MeOH). RMN ($CDCl_3$) δ : 0,96 (d, J = 7 Hz, 3H, Me) 1,08 (d, J = 7 Hz, 3H, Me) 1,36 (s, 9H, Boc) 2,09 (m, 1H, $CH^{\beta}$) 3,11 (s1, 1H, $CH^{\alpha}$) 3,91 (m, 2H, $CH_2$ Bzl) 7,24-7,37 (m, 5H, arom) 10,21 (s1, 1H, $CO_2H$).
*Analyse élémentaire* $C_{17}H_{26}N_2O_4$ calc (%) C 63,33 ; H 8,13 ; N 8,69 ; trouvé C 63,23 ; H 8,10 ; N 8,70.

Exemple 3 : Préparation de N$^{\alpha}$-benzyl-N$^{\beta}$-Boc-(L)-hydrazinoisoleucine (1c)

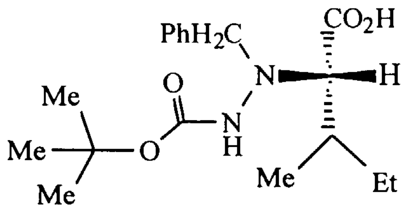

(L)-(1c)

Selon le mode opératoire type, en partant de 222 mg (1 mmol) de N-benzyl-(L)-isoleucine, on obtient 260 mg (78%) de (1c), sous la forme d'un solide, F 108 °C (décomposition); $[\alpha]_D^{25}$ +32,7 (c = 1,00 ; MeOH) ; RMN ($CDCl_3$) δ : 0,83 (t, J = 7 Hz, 3H, $Me^{\delta}$) 0,92 (d, J = 7 Hz, 3H, Me) 1,35 (m, 10H, Boc + $CH^{\gamma}$) 1,86 (m, 2H, $CH^{\beta}$ + $CH^{\gamma}$) 3,23 (sl, 1H, $CH^{\alpha}$) 3,91 (m, 2H, $CH_2$ Bzl) 7,24-7,35 (m, 5H, arom).
*Analyse élémentaire* $C_{18}H_{28}N_2O_4$ calc. (%) C 64,26 ; H 8,39 ; N 8,33 ; trouvé C 64,18 ; H 8,34 ; N 8,54.

Exemple 4 : Préparation de N$^{\alpha}$-benzyl-N$^{\beta}$-Boc-(L)-hydrazinosérine (1d)

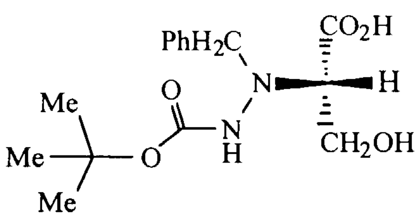

(L)-(1d)

On suit le mode opératoire type en partant de 195 mg (1 mmol) de N-benzyl-(L)-sérine. On obtient 210 mg (68%) de (1d). Solide, F 141 °C (décomposition) ; $[\alpha]_D^{25}$ +9,0 (c = 1,07 ; MeOH) ; RMN ($CDCl_3$) δ : 1,30 (s, 9H, Boc) 3,78-4,13 (m, 5H, $CH_2^{\beta}$ + $CH^{\alpha}$ + $CH_2$ Bzl) 6,37 (s, 1H, NH) 7,24-7,36 (m, 5H, arom).
*Analyse élémentaire* [$C_{15}H_{22}N_2O_5$ + 0,25 $H_2O$] calc. (%) C 57,22 ; H 7,20 ; N 8,90 ; trouvé C 57,02 ; H 6,94 ; N 8,80.

Exemple 5 : Préparation de $N^{\alpha}$-benzyl-$N^{\beta}$-Boc-(L)-hydrazinoasparagine (1e)

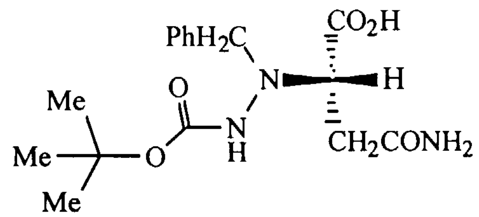

(L)-(1e)

Selon le mode opératoire type, en partant de 444 mg (2 mmol) de N-benzyl-(L)-asparagine, on obtient 440 mg (65%) de (1e). F 189 °C (décomposition) ; $[\alpha]_D^{25}$ -17,2 (c =1,10 ; MeOH) ; RMN ($CDCl_3$) $\delta$ : 1,28 (s, 9H, Boc) 2,70 (d, J = 7 Hz, 2H, $CH_2^{\beta}$) 3,82 (t, J = 7Hz, 1H, $CH^{\alpha}$) 4,09 (q, J = 8 Hz, 2H, $CH_2$ Bzl) 6,22 et 6,67 (s, 2H, $NH_2$) 7,24-7,32 (m, 5H, arom). *Analyse élémentaire* $C_{16}H_{23}N_3O_5$ calc. (%) C 56,96; H 6,87 ;N12,46 ; trouvé C 56,75 ; H 7,09 ;N 12,28.

Exemple 6 : Préparation de $N^{\alpha}$-benzyl-$N^{\beta}$-Boc-(L)-hydrazinotryptophane (1f)

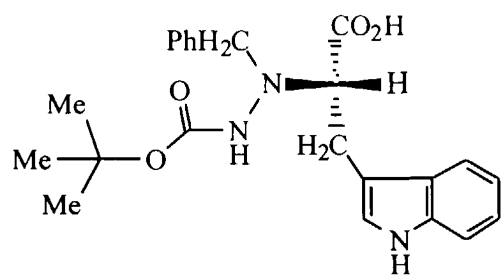

(L)-(1f)

Selon le mode opératoire type, 588 mg (2 mmol) de N-benzyl-(L)-tryptophane conduisent à 670 mg (80%) de (1f), solide se décomposant par chauffage ; $[\alpha]_D^{25}$ +1,6 (c = 1,17 ; MeOH) ; RMN ($CDCl_3$) $\delta$ : 1,37 (s, 9H, Boc) 3,23-3,54 (m, 2H, $CH_2^{\beta}$ ) 3,81 et 4,01 (m, 3H, CHa + $CH_2$ Bzl) 6,45 (si, 1H, NH) 7,07-7,57 (m, 10H, arom) 8,42 (s1, 1H, NH indole) 10,15 (s1, 1H, $CO_2H$).
*Analyse élémentaire* [$C_{23}H_{27}N_3O_4$ + 0,25 $H_2O$] calc. (%) C 66,73 ; H 6,70 ; N 10,15 ; trouvé C 67,00 ; H 6,83 ; N 9,83.

Exemple 7 : Préparation de $N^{\alpha}$-benzyl-$N^{\beta}$-Boc-(L)-hydrazinoaspartate de benzyle (1g)

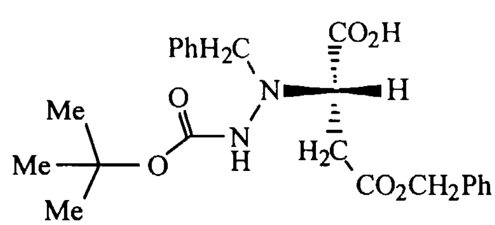

(L)-(1g)

Selon le mode opératoire type, 313 mg (1 mmol) de $N^{\alpha}$-benzyl-β-(L)-aspartate de benzyle donnent 318 mg (75%) de (1g). Solide se décomposant à 113 °C ; $[\alpha]_D^{25}$ -4,2 (c = 0,98 ; MeOH) ; RMN ($CDCl_3$) $\delta$ : 1,26 (s, 9H, Boc) 2,86 (m, 2H, $CH_2^{\beta}$ ) 3,90 et 4,02 (m, 3H, CHa + $CH_2$ Bzl) 5,14 (s, 2H, $OCH_2$) 6,15 (s1, 1H, NH) 7,25 (s, 5H, arom) 7,32

(s, 5H, arom).
*Analyse élémentaire* $C_{23}H_{28}N_2O_6$ calc. (%) C 64,47 ; H 6,59; N 6,54 ; trouvé C 64,31 ; H 6,58 ; N 6,58.

Exemple 8 : Préparation de $N^{\alpha}$-benzyl-$N^{\beta}$-Boc-$N^{\varepsilon}$-Z-(L)-hydrazinolysine (1h)

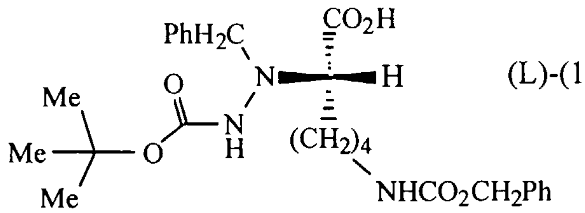

(L)-(1h)

Selon le mode opératoire type, 375 mg (1 mmol) de $N^{\alpha}$-benzyl-$N^{\varepsilon}$-carbobenzoxy-(L)-lysine conduisent à l'isolement de (1h) sous la forme d'une huile hygroscopique que l'on transforme en son sel de sodium en le traitant par 1,5 mmol de NaOH dans 5 ml d'eau. Après lyophilisation, on obtient 365 mg (72%) du sel de sodium de (1h). $[\alpha]_D^{25}$ +35,1 (c = 0,99 ; MeOH) ; RMN ($D_2O$) δ : 0,98 et 1,10 (s, 9H, Boc) 1,32 (m, 4H, $CH_2^{\gamma\delta}$) 1,49 (m, 2H, $CH_2^{\beta}$) 2,97 (m, 2H, $CH_2^{\varepsilon}$) 3,12 (m, 1H, $CH^{\alpha}$) 3,66 (m, 2H, $CH_2$ Bzl) 4,96 (s, 2H, OCH2) 7,24 (s, 5H, arom) 7,28 (s, 5H, arom).

Exemple 9 : Préparation de $N^{\alpha}$-benzyl-$N^{\beta}$-Boc-(L)-hydrazinométhionine-S-oxyde (1i)

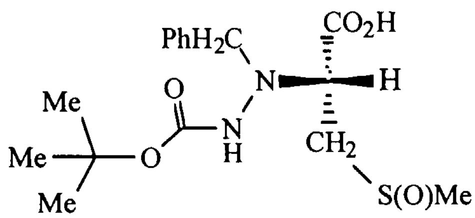

(L)-(1i)

Selon le mode opératoire type, en partant de 255 mg (1 mmol) du sulfoxyde de la N-benzyl-(L)-méthionine, on obtient 281 mg (76%) du composé (1i). Solide se décomposant par chauffage ; $[\alpha]_D^{25}$ +9,7 (c = 1,03 ; MeOH) ; RMN ($CDCl_3$) δ : 1,33 (s, 9H, Boc) 2,08-2,28 (m, 2H, $CH_2^{\beta}$) 2,59 et 2,62 (s, 3H, SOMe) 3,10 (m, 1H, $CH^{\alpha}$) 3,44 (m, 2H, $CH^{\gamma}$) 4,02 (m, 2H, $CH_2$ Bzl) 6,86 (sl, 1H, NH) 7,24-7,34 (m, 5H, arom).
*Analyse élémentaire* $C_{17}H_{26}N_2O_5S$ calc. (%) C 55,12 ; H 7,07 ; N 7,56 ; S 8,65 ; trouvé C 54,87 ; H 7,00 ; N 7,84 ; S 8,22.

Exemple 10 : Préparation de N$^{\alpha}$-benzyl-N$^{\beta}$-Boc-O-benzyl-(L)-hydrazinotyrosine (1j)

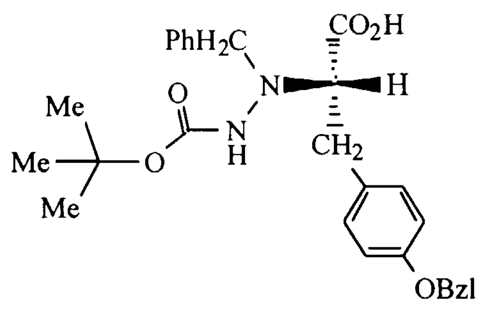

(L)-(1j)

Selon le mode opératoire type, en partant de 366 mg (1 mmol) de l'éther de benzyle de la N$^{\alpha}$-benzyl-(L)-tyrosine, on obtient 250 mg (52%) de (1j). Solide se décomposant par chauffage ; $[\alpha]_D^{25}$ +13,3 (c = 1,35 ; MeOH) ; RMN ($CDCl_3$) δ : 1,33 (s, 9H, Boc) 2,98 et 3,29 (m, 2H, $CH_2^{\beta}$) 3,70 et 3,83 (m, 3H, $CH^{\alpha}$ + $CH_2$ Bzl) 5,06 (s, 2H, $OCH_2$) 5,88 (sl, 1H, NH) 6,94 (m, 2H, arom) 7,17-7,41 (m, 12H, arom).
*Analyse élémentaire* [$C_{28}H_{32}N_2O_5$ + 0,25 $H_2O$] calc. (%) C 69,91 ; H 6,81 ; N 5,82 ; trouvé C 69,79 ; H 6,92 ; N 5,83.

Exemple 11 : Préparation de N$^{\alpha}$-benzyl-N$^{im}$-benzyl-N$^{\beta}$-Boc-(L)-hydrazinohistidine (1k)

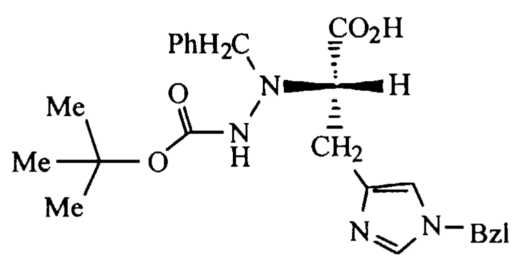

(L)-(1k)

Selon le mode opératoire type, en partant de 359 mg (1 mmol) de N$^{\alpha}$-benzyl-N$^{im}$-Benzyl-(L)-histidine, on obtient 338 mg (75%) de (1k) sous forme d'une huile jaune qui se solidifie dans le pentane. $[\alpha]_D^{25}$ -34,2 (c = 1,33 ; MeOH) ; RMN ($CDCl_3$) δ : 1,31 (s, 9H, Boc) 3,07 (m, 2H, $CH_2^{\beta}$) 3,55 (m, 3H, $CH^{\alpha}$) + 3,97 (m, 2H, $CH_2$ Bzl) 5,08 (m, 2H, $CH_2$ Bzl imidazole) 6,96-7,34 (m, 12H, arom).
*Analyse élémentaire* [$C_{34}H_{41}N_5O_8$ + 0,75 $H_2O$] calc. (%) C 64,71 ; H 6,84 ; N 12,07 ; trouvé C 64,67; H 6,87 ; N 11,92.

Exemple 12: Préparation de N$^{\alpha}$-benzyl-N$^{\beta}$-Fmoc-(L)-hydrazinoalanine (2)

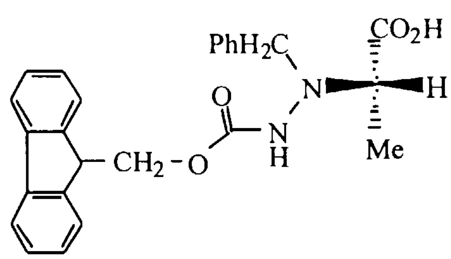

(L)-(2)

On traite 164 mg (0,556 mmol) de N$^{\alpha}$-benzyl-N$^{\beta}$-Boc-(L)-hydrazinoalanine par 1 ml d'une solution 3 M de

chlorure d'hydrogène dans l'acétate d'éthyle. Après 2 heures d'agitation à température ambiante le mélange est soigneusement concentré sous vide puis repris par 1,45 ml d'eau et 0,6 ml de dioxane. On traite à 0 °C par 554 mg (1,93 mmol) de carbonate de sodium décahydrate puis par 156 mg (0,58 mmol) de chloroformiate de 9-fluorénylméthyle à 97%. On agite ensuite une nuit à température ambiante. Le mélange réactionnel est filtré et le solide lavé par 2 ml d'un mélange d'eau et de dioxane. Le filtrat est concentré puis repris par 15 ml d'une solution à 10% de NaCl. La phase aqueuse est lavée deux fois par 10 ml d'éther puis acidifiée jusqu'à pH 3 par de l'acide chlorhydrique 6 M. Après extraction par deux fois 10 ml d'éther, séchage de la phase organique sur $Na_2SO_4$ et évaporation, on recueille 140 mg (61%) de $N^{\alpha}$-benzyl-$N^{\beta}$-Fmoc-(L)-hydrazinoalanine sous forme d'un solide blanchâtre pur en CCM.
Caractéristiques physiques. Décomp. vers 60 °C ; $[\alpha]_D^{25}$ 13,9 (c=0,5, MeOH); $^1$H RMN (DMSO-d6) δ : 1,03 (d, 3H, CH3, J=5,8 Hz) ; 3,47-4,37 (m, 6H); 7,20-7,89 (m, 13 H) ; 8,17 (s, 1H, NH) ; 12,54 (s, 1H, $CO_2H$).
*Analyse élémentaire* $C_{25}H_{24}N_2O_4$.

À partir des acides hydrazinés protégés des exemples 1 et 3, on va décrire la préparation d'hydrazinopeptides.

Exemple 13 : Préparation de Boc(Bzl)hAlaAlaNHiPr (3)

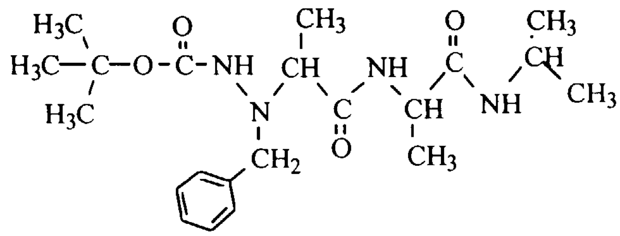

(3)

1) Préparation de l'ester activé. Boc(Bzl)hAla (0,5 mmol, 151 mg) est placé à 0 °C dans 0,5 ml de DMF sec en présence de DCC (0,5 mmol, 103 mg) et de pentafluorophénol (0,5 mmol, 92 mg). Le milieu réactionnel est agité pendant 15 heures dans le réfrigérateur.
2) Couplage. La solution d'ester activé ci-dessus est filtrée directement sur HAlaNHiPr (0,5 mmol, 65 mg), la DCU filtrée est rincée par 0,1 ml de DMF puis le milieu est agité 24 heures à température ambiante. Après évaporation du DMF, le produit brut est repris par 2 ml de $CH_2Cl_2$ et lavé par trois fois 2 ml d'eau. La phase organique est séchée, évaporée et le résidu chromatographié sur une colonne de 5 g de silice (éluant $CH_2Cl_2$/MeOH 50/50). On recueille 136 mg (67%) d'une fraction pure de l'hydrazinodipeptide protégé Boc(Bzl)hAlaAlaNHiPr (3). $[\alpha]_D^{25}$ - 21,1 (c = 1; $CH_2Cl_2$); RMN ($CDCl_3$) δ : 1,0 à 1,22 (m, 21H, $CH_3$ BOC + $CH_3$ Ala + $CH_3$ iPr) 3,87 (s, 2H, $CH_2$-Ph) 3,97 (q, J = 6,8Hz, 1H, *NH-N(Bzl)*-C<u>H</u>-) 4,32 (m, 1H, CHa Ala) 6,19 (d, J = 7,4Hz, 1H, NH) 7,30 (m, 5H, Ph) 8,43 (s, 1H, NH).

*Analyse élémentaire* [$C_{21}H_{34}N_4O_4$ + 0,75 $H_2O$] calc (%) C 60,00 ;H 8,51 ; N 13,34; trouvé C 59,91; H 8,11 ; N 13,22.

Exemple 14 : Préparation de BocVal(Bzl)hAlaAlaNHiPr (4)

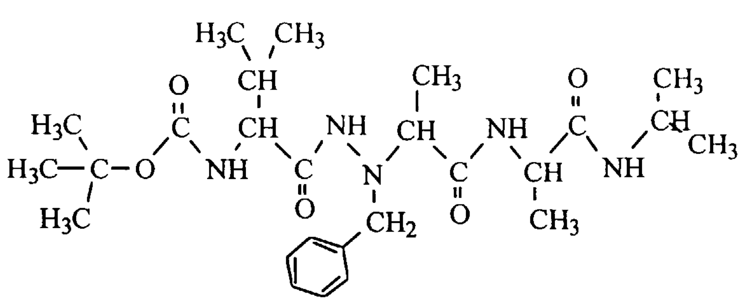

(4)

On place BocValNCA. (0,46 mmol, 99 mg) dans 1 ml de THF sec sous argon à température ambiante. Le

chlorhydrate de (Bzl)hAlaAlaNHiPr (0,46 mmol, 155 mg) obtenu par traitement de Boc(Bzl)hAlaAlaNHiPr (0,46 mmol, 193 mg) par HCl 3 M dans l'acétate d'éthyle pendant une nuit puis évaporation est ajouté en solution dans 5 ml de THF sec, en présence de triéthylamine séchée sur tamis (0,46 mmol, 64 µl). Le mélange est agité 2 heures à température ambiante. Après évaporation du THF, le résidu est repris par 5 ml de $CH_2Cl_2$ puis lavé par 5 ml d'eau, et 5 ml d'une solution de $KHCO_3$ à 5%. On obtient après séchage et évaporation, 225 mg de produit qui est purifié par chromatographie sur colonne de 7 g de silice (éluant CH2Cl2/MeOH 95/5). On recueille 140 mg (60%) de produit pur (4) sous forme d'une poudre blanche. $[\alpha]_D^{25}$ +1,3 (c = 0,75 ; $CH_2Cl_2$); RMN (DMSO) δ : 0,56 (d, J = 6,5Hz, 3H, $CH_3$ iPr ou $CH_3$ Val) 0,66 (d, J = 6,5Hz, 3H, $CH_3$ iPr ou $CH_3$ Val) 1,0 (d, J = 6,5Hz, 3H, $CH_3$ iPr ou $CH_3$ Val) 1,04 (d, J = 6,5Hz, 3H, $CH_3$ iPr ou $CH_3$ Val) 1,34 (s, 9H, $CH_3$ Boc) 1,45 (d, J = 6,4 Hz, 3H, $CH_3$ Ala) 1,66 (m, 1 H, CHβ Val) 1,75 (d, J = 6Hz, 3H, $CH_3$ Ala) 3,3 à 3,6 (m, 2H, CHα) 4,18 (m, 1H, CHα) 4,85 (m, 3H, $CH_2$-Ph + CHα) 6,58 (d, J =8,4 Hz, 1H, NH-Boc) 7,3 (m, 5H, Ph) 7,66 (d, J = 8Hz, 1H, NH Ala ou NH-iPr) 8,46 (d, J = 8Hz, 1H, NH Ala ou NH-iPr) 8,97 (s, 1H, NH-N).
*Analyse élémentaire* [$C_{26}H_{43}N_5O_5$ + 1 $H_2O$] calc. (%) C 59,74; H 8,57; N 13,39 ; trouvé C 59,50 ; H 8,57; N 12,91.

Exemple15 : Préparation de Boc(Bzl)hIleLeuOMe (5)

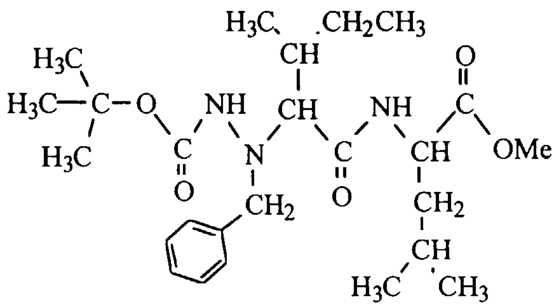

(5)

Un mélange de 84,1 mg (0,25 mmol) de Boc(Bzl)hIle et de 54,5 mg (0,30 mmol) de chlorhydrate de LeuOMe en solution dans 0,5 ml de chlorure de méthylène est traitée à 0 °C par 130 mg (0,25 mmol) de PyBop puis par 105 µl de triéthyamine (0,75 mmol). Le mélange est agité 10 minutes à 0 °C puis porté à température ambiante pendant une nuit. Après évaporation du solvant et chromatographie sur 10 g de gel de silice (éluant $CH_2Cl_2$/MeOH 98/2), on isole 83,4 mg (72%) de dipeptide Boc(Bzl)hIleLeuOMe (5) qui cristallise au bout de quelques semaines. F 74 °C ; $[\alpha]_D^{25}$ +13,9 (c = 1 ; MeOH) ; RMN (DMSO) δ : 0,76-0,93 (m, 12H, $CH_3$) 1,19-1,28 (m, 10H, Boc et CH ) 1,45-1,91 (m, 5H, CH Ile et Leu) 3,03 (d, 1H, CHa hile, J = 8,7Hz) 3,61 (s, 3H, OMe) µ3,77 (s large, 2H, $CH_2$Ph) 4,35 (s large, 1H, CHa Leu) 7,06-7,32 (m, 5H, Ph) 7,70 (s large, 1H, NHBoc) 8,57 (d,lH, NH, J = 6,5 Hz).
*Analyse élémentaire* $C_{25}H_{41}N_3O_5$ calc (%) C 64,77 ; H 8,91 ; N 9,06 ; trouvé C 64,61 ; H 8,96 ; N 9,09.

Exemple16 : Préparation de BocValhIleLeuOMe (6)

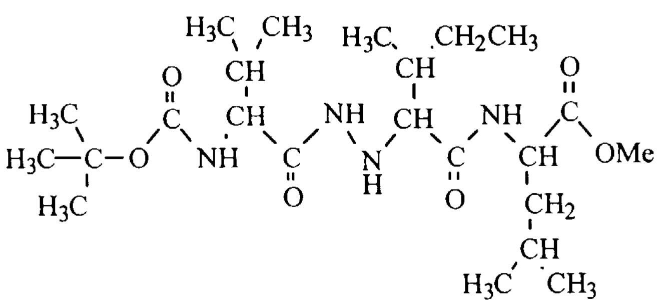

(6)

1) Déprotection de (6). Une suspension de 5 mg de palladium à 5 % sur charbon et de 80 mg (0,172 mmol) de Boc(Bzl)hIleLeuOMe (5) dans 2 ml de méthanol est hydrogénée à pression atmosphérique pendant 1 heure. Le

produit obtenu après filtration sur célite puis concentration est ensuite traité par 0,5 ml de HCl 3 M dans l'acétate d'éthyle pendant une nuit, pour conduire après concentration à 54 mg (100%) de chlorhydrate de hIleLeuOMe.
2) Couplage. Une solution portée à -15 °C de 37,7 mg de BocVal dans 1ml de THF anhydre est traitée par 19 µl (0,172 mmol) de N-méthylmorpholine puis par 22 µl de chlorofomiate d'isobutyle. Après 3 minutes d'agitation, une solution de 54 mg de chlorhydrate de hIleLeuOMe, 20 µl de N-méthylmorpholine dans 0,5 ml de DMF anhydre est introduite. On laisse la température remonter lentement et après une nuit on concentre. Le résidu est repris par 15 ml de dichlorométhane puis lavé par de l'hydrogénosulfate de sodium à 10%, par de l'hydrogénocarbonate de sodium à 10% puis par de l'eau. Après séchage sur sulfate de sodium, concentration et chromatographie sur 2,5 g de silice (éluant $CH_2Cl_2/Et_2O$ 80/20), on isole 54 mg (67%) de BocValhIleLeuOMe (6) sous forme d'un solide blanc. $[\alpha]_D^{25}$ -69,9 (c =0,6 ; MeOH) ; RMN (DMSO) δ : 0,77-0,90 (m, 18H, $CH_3$) 1,19 (m, 2H, CH) 1,36 (s, 9H, Boc) 1,47-1,85 (m, 5H, CH Ile,Val et Leu) 3,22 (dd, 1H, CHα hIle, J = 4,2Hz) 3,59 (s, 3H, OMe) 3,71(m, 1H, CHα Val) 4,25 (s large, 1H, CHα Leu) 5,00 (s large, 1H, NHa) 6,52 (d, 1H, NH Val, J = 8,8 Hz) 8,15 (d, 1H, NH Leu, J = 7,3 Hz) 9,12 (s large, 1H, NH hIle).

*Analyse élémentaire* $C_{23}H_{44}N_4O_6$ calc. (%) C58,45 ; H 9,38 ; N 11,85 ; trouvé C 58,10 ; H 9,37; N 11,60.

Les nouveaux composés conformes à l'invention se caractérisent en ce qu'ils comportent des groupes protecteurs orthogonaux, et peuvent ainsi être utilisés comme synthons pour la préparation d'une nouvelle classe de pseudopeptides d'intérêt thérapeutique, sans modification des techniques de synthèse peptidique en usage, notamment dans les synthétiseurs automatiques.

Comme déjà dit, leur préparation est aisée à partir de dérivés simples d'acides aminés. Certaines des oxaziridines utilisables pour leur synthèse sont elles-mêmes accessibles par des voies économiquement viables.

La généralisation de l'invention comme nouvelle méthode d'accès aux α-hydrazinoacides énantiomériquement purs ou racémiques, par simple clivage des deux groupes protecteurs $R_4$ et $R_5$ présente un grand intérêt, car elle permet d'obtenir facilement de tels composés avec des chaînes latérales très variées. Il suffit de disposer des acides α-aminés correspondants.

En résumé, ces synthons peuvent être utilisés avantageusement pour :

- la synthèse de pseudopeptides de la famille des hydrazinopeptides ou des N-aminopeptides par les procédés courants utilisant les méthodologies Boc ou Fmoc, y compris dans les synthétiseurs automatiques ;
- la synthèse combinatoire de pseudopeptides ;
- la synthèse d'a-hydrazinoacides L, D ou DL.

## Revendications

1. Dérivé d'α-hydrazinoacide de formule générale :

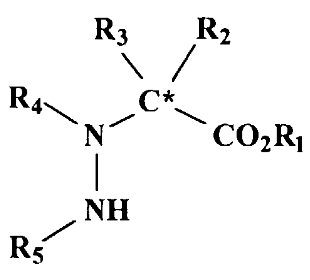

dans laquelle :

. $R_1$, $R_2$, $R_3$ désignent l'hydrogène ou un radical carboné,
. lorsque $R_2$ et $R_3$ sont différents, C* désigne un carbone asymétrique de configuration L, D ou DL,
. $R_4$ et $R_5$ désignent un groupe protecteur,

***caractérisé :***

- en ce que $R_4$ désigne un radical benzyle $ArCH_2$

- . où Ar désigne un radical phényle ou phényle substitué par un ou plusieurs groupes X ;
- . où X désigne un halogène, un radical nitro ou un radical alkyle ;

- et en ce que $R_5$ désigne un groupe Y-O-CO, dans lequel Y désigne un radical carboné différent de $R_4$.

2. Dérivé d'α-hydrazinoacide selon la revendication 1, ***caractérisé*** en ce que :

- $R_1$ est l'hydrogène ou un groupe alkyle ou benzyle ;
- $R_2$ désigne l'hydrogène ou un groupe alkyle ;
- $R_3$ désigne l'hydrogène, une chaîne latérale (protégée ou non) des acides aminés protéogéniques naturels, un groupe aromatique, tel que le phényle ou le para-hydroxyphényle, un alkyle primaire, secondaire ou tertiaire.

3. Dérivé d'α-hydrazinoacide selon la revendication 2, ***caractérisé*** en ce que $R_3$ est une chaîne latérale (éventuellement protégée) des acides aminés protéogéniques.

4. Dérivé d'a-hydrazinoacide selon la revendication 3, ***caractérisé*** en ce que la chaîne latérale $R_3$ est protégée.

5. Dérivé d'α-hydrazinoacide selon la revendication 3, ***caractérisé*** en ce que $R_5$ est un radical terbutoxycarbonyle (Boc).

6. Dérivé d'α-hydrazinoacide selon la revendication 3, ***caractérisé*** en ce que $R_5$ est un radical 9-fluorénylméthoxycarbonyle (Fmoc).

7. Dérivé d'α-hydrazinoacide selon l'une des revendications 1 à 5, ***caractérisé*** en ce que dans $R_4$ Ar est égal à phényle.

8. Procédé pour la préparation d'un dérivé d'α-hydrazinoacide selon l'une des revendications 5 à 6, ***caractérisé*** en ce que ce composé est obtenu de façon directe par réaction des acides α-aminés-N-benzylés correspondants avec un dérivé de la 3-phényloxaziridine (éventuellement substitué sur le phényle par un ou plusieurs groupes tels que CN, halogène) dont l'azote porte un groupe terbutoxycarbonyle (Boc) ou 9-fluorénylméthoxycarbonyle (Fmoc).

9. Procédé pour la préparation d'un dérivé d'α-hydrazinoacide selon la revendication 6, ***caractérisé*** en ce que ce composé est obtenu de façon indirecte à partir d'un dérivé de la revendication 5 par déprotection sélective du groupe Boc suivie de reprotection de l'azote libéré par réaction avec Fmoc-Cl.

## Claims

1. α-Hydrazino acid derivative of general formula:

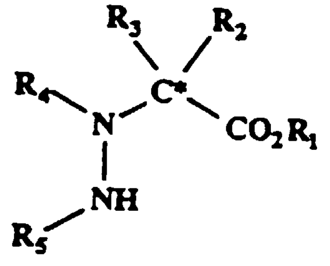

in which:

- . $R_1$, $R_2$ and $R_3$ denote hydrogen or a carbonaceous radical,
- . while $R_2$ and $R_3$ are different, C* denotes an asymmetric carbon of L, D or DL configuration,
- . $R_4$ and $R_5$ denote a protecting group,

characterized:

- in that $R_4$ denotes a benzyl radical $ArCH_2$
  - . where Ar denotes a phenyl radical or a phenyl radical substituted with one or more groups X;
  - . where X denotes a halogen, a nitro radical or an alkyl radical;
- and in that $R_5$ denotes a group Y-O-CO, in which Y denotes a carbonaceous radical different from $R_4$.

**2.** α-Hydrazino acid derivative according to Claim 1, characterized in that

- $R_1$ is hydrogen or an alkyl or benzyl group;
- $R_2$ denotes hydrogen or an alkyl group;
- $R_3$ denotes hydrogen or a side chain (protected or otherwise) of natural proteogenic amino acids, an aromatic group such as phenyl or para-hydroxyphenyl, a primary, secondary or tertiary alkyl.

**3.** α-Hydrazino acid derivative according to Claim 2, characterized in that $R_3$ is a side chain (optionally protected) of proteogenic amino acids.

**4.** α-Hydrazino acid derivative according to Claim 3, characterized in that the side chain $R_3$ is protected.

**5.** α-Hydrazino acid derivative according to Claim 3, characterized in that $R_5$ is a tert-butoxycarbonyl (Boc) radical.

**6.** α-Hydrazino acid derivative according to Claim 3, characterized in that $R_5$ is a 9-fluorenylmethoxycarbonyl (Fmoc) radical.

**7.** α-Hydrazino acid derivative according to one of Claims 1 to 5, characterized in that in $R_4$, Ar is equal to phenyl.

**8.** Method for preparing an α-hydrazino acid derivative according to either of Claims 5 and 6, characterized in that this compound is obtained directly by reacting the corresponding N-benzylated α-amino acids with a derivative of 3-phenyloxaziridine (optionally substituted on the phenyl with one or more groups such as CN, halogen) in which the nitrogen carries a tert-butoxycarbonyl (Boc) or 9-fluorenylmethoxycarbonyl (Fmoc) group.

**9.** Method for preparing an α-hydrazino acid derivative according to Claim 6, characterized in that this compound is obtained indirectly from a derivative of Claim 5 by selective deprotection of the Boc group followed by reprotection of the nitrogen liberated by reacting with Fmoc-Cl.

**Patentansprüche**

**1.** α-Hydrazinsäurederivat der allgemeinen Formel

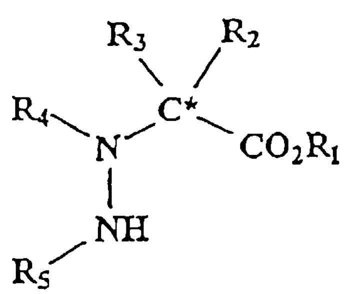

in der $R_1$, $R_2$, $R_3$ Wasserstoff oder ein Kohlenstoffradikal sind,
wenn er $R_2$ und $R_3$ verschieden sind, C* ein asymmetrisches Kohlenstoffatom der L, D oder DL Konfiguration ist,
$R_4$ und $R_5$ Schutzgruppen sind, dadurch gekennzeichnet, daß:
$R_4$ ein $ArCH_2$-Benzylradikal

oder Ar ein Phenylradikal oder mit einer oder verschiedenen Gruppen X substituiertes Phenylradikal, oder X ein Halogen, ein Nitroradikal oder ein Alkylradikal sind,
und wobei $R_5$ eine Y-O-CO-Gruppe isc, in der Y ein anderes Kohlenstoffradikal als $R_4$ ist.

**2.** α-Hydrazinsäurederivat nach Anspruch 1, dadurch gekennzeichnet, daß

$R_1$ Wasserstoff oder eine Alkyl- oder benzylgruppe,
$R_2$ Wasserstoff oder eine Alkylgruppe,
$R_3$ Wasserstoff, eine geschützte oder ungeschützte Seitenkette von natürlichen proteogenen Aminosäuren, eine aromatische Gruppe wie eine Phenylgruppe oder eine para-Hydroxyphenylgruppe, ein primäres, sekundäres oder tertiäres Alkyl ist.

**3.** α-Hydrazinsäurederivat nach Anspruch 2, dadurch gekennzeichnet, daß $R_3$ eine seitenketce (gegebenenfalls geschützt) von proteogenen Aminosäuren ist.

**4.** α-Hydrazinsäurederviat nach Anspruch 3, dadurch gekennzeichnet, daß die Seitenkette $R_3$ geschützt ist.

**5.** α-Hydrazinsäurederivat nach Anspruch 3, dadurch gekennzeichnet, daß $R_5$ eine ter-Butoxycarbonyl(Boc)-gruppe ist.

**6.** α-Hydrazinsäurederivat nach Anspruch 3, dadurch gekennzeichnet, daß $R_5$ eine 9-Fluorenylmethoxycarbonyl (Fmoc)-gruppe ist.

**7.** α-Hydrazinsäurederivat nach einem der Ansprüche 1 bis 5, dadurch gekennzeichnet, daß Ar in $R_4$ eine Phenylgruppe ist.

**8.** Verfahren zur Herstellung eines α-Hydrazinsäurederivats gemäß einem der Ansprüche 5 und 6, dadurch gekennzeichnet, daß man es direkt durch Umsetzung von entsprechenden α-aminierten-N-benzylierten Säuren mit einem Derivat von 3-Phenyloxaziridin (gegebenenfalls am Phenyl Eubstituiert durch eine oder mehrere Gruppen wie CN, Halogen), dessen Stickstoff eine ter-Butoxycarbonyl(Boc)- oder eine 9-Fluorenylmethoxycarbonyl(Fmoc)-Gruppe, erhält.

**9.** Verfahren zur Herstellung eines α-Hydrazinsäurederivats nach Anspruch 6, dadurch gekennzeichnet, daß man es indirekt aus einem Derivat gemäß Anspruch 5 durch selektive Deprotektionierung der Boc-Gruppe, gefolgt von der Reprotektionierung des freien Stickstoffs durch Umsetzung mit Fmoc-Cl, erhält.